# EUROPEAN PATENT SPECIFICATION

(11) **EP 2 809 357 B1**
(45) Date of publication and mention of the grant of the patent: **09.08.2017**
(21) Application number: 13716031.3
(22) Date of filing: 29.01.2013
(51) Int. Cl.: A61K 51/04, A61K 31/519, A61K 31/7076

(54) **A METHOD OF USING ADENOSINE AND DIPYRIDAMOLE FOR PHARMACOLOGIC STRESS TESTING, WITH SPECIFIC COMPOSITIONS, UNIT DOSAGE FORMS AND KITS**
VERFAHREN ZUR VERWENDUNG VON ADENOSIN UND DIPYRIDAMOL ZUR PRÜFUNG VON PHARMAKOLOGISCHEM STRESS MIT SPEZIFISCHEN ZUSAMMENSETZUNGEN, EINHEITSDOSIERUNGSFORMEN UND KITS
PROCÉDÉ D'UTILISATION DE L'ADÉNOSINE ET DU DIPYRIDAMOLE DANS DES ÉPREUVES DE STRESS PHARMACOLOGIQUE, À L'AIDE DE COMPOSITIONS SPÉCIFIQUES, DE FORMES PHARMACEUTIQUES UNITAIRES ET DE TROUSSES

(30) Priority: 03.02.2012 US 201261594744 P; 11.06.2012 US 201261658025 P; 11.06.2012 US 201261658019 P
(43) Date of publication of application: 10.12.2014
(73) Proprietor: Adenobio N.V., 75016 Paris (FR)
(72) Inventor: GORNY, Philippe, F-75016 Paris (FR)
(74) Representative: J A Kemp
(86) International application number: PCT/IB2013/000332
(87) International publication number: WO 2013/114204

(56) References cited:
- WO-A1-2008/003479
- WO-A2-01/07089
- WO-A2-2007/010534
- MAHMARIAN J J ET AL: "Myocardial perfusion imaging during pharmacologic stress testing", CARDIOLOGY CLINICS, W.B. SAUNDERS COMPANY, PHILADELPHIA, US, vol. 12, no. 2, 1 January 1994 (1994-01-01), pages 223-245, XP009090182, ISSN: 0733-8651
- CONRADSON T B G ET AL: "Cardiovascular effects of infused adenosine in man: potentiation by dipyridamole", ACTA PHYSIOLOGICA SCANDINAVICA, OXFORD, GB, vol. 129, no. 3, 1 January 1987 (1987-01-01), pages 387-391, XP009090152,

## Description

The present invention relates to a fixed dose of dipyridamole of from 9 mg to 14 mg and a fixed dose of adenosine of from 1 mg to 4 mg for use in a method of performing cardiac diagnosis as defined in the claims, and a composition in dry or fluid form comprising said fixed doses in unit dosage form, as defined in the claims.

### BACKGROUND

Various compositions and methods for functional assessment of myocardial perfusion have been described but are not optimal.

### SUMMARY

Described herein are exemplary methods, dosages, compositions, concentrations, unit dosage forms (UDFs) and kits that allow adenosine and dipyridamole to be used in combination so as to meet the elements defined in the following paragraphs.

According to the invention, the use in the method of performing cardiac diagnosis comprises administering to a patient in need thereof a fixed dose of dipyridamole and a fixed dose of adenosine, wherein the fixed doses of dipyridamole and adenosine are independent of patient weight, and both dipyridamole and adenosine are parenterally injected as a slow bolus, as defined in the claims.

In an embodiment, the method described above is used to perform hemodynamic or cardio-electric measurements under stress conditions using transthoracic doppler-echocardiography or transesophageal doppler echography and/or electrocardiography techniques.

In an exemplary embodiment, the method described above comprises administering to a patient in need thereof a fixed dose of dipyridamole and a fixed dose of adenosine as defined in the claims, and also administering to said patient an imaging agent in the form of a radionuclide, a radiopharmaceutical or a contrast agent using either nuclear techniques, such as SPECT or PET-scanning, two and three dimensional ultrasound techniques, such as Echography, and real-time myocardial contrast echocardiography (MCE), nuclear magnetic resonance (NMR) imaging or CT-scan imaging techniques.

In an exemplary method of reducing radiation and total study time to a patient undergoing cardiac diagnosis using a nuclear technique, such as single photon emission computed tomography (SPECT), especially new SPECT technologies performed with ultrafast gamma-cameras, as described below, the fixed doses of dipyridamole and adenosine are administered as a bolus over a total period of about 30 seconds, the imaging agent is then administered over a period of less than about 10 seconds, and imaging of the patient is performed for about 4 minutes to about 5 minutes after the administration of the imaging agent.

In an exemplary method of reducing radiation exposure and total study time to a patient undergoing cardiac diagnosis using a nuclear technique such as single photon emission computed tomography (SPECT), dipyridamole and adenosine are administered as a bolus over a total period of about 30 seconds, the imaging agent is then administered over a period of less than about 10 seconds, imaging of the patient is performed for about 3 minutes after the administration of the imaging agent; a second bolus administration of dipyridamole and adenosine at a reduced dosage is made about 3 minutes to about 4 minutes after the initial administration, and imaging of the patient is performed for about 2 minutes after the second administration of dipyridamole and adenosine.

In an exemplary method of reducing radiation exposure and the duration of the full stress-rest or rest-stress protocol to a patient undergoing cardiac diagnosis using a nuclear technique such as single photon emission computed tomography (SPECT), especially new SPECT technologies performed with ultrafast gamma-cameras as described below, the method comprises administering parenterally to a patient in need thereof a fixed dose of dipyridamole and a fixed dose of adenosine injected as a slow bolus and administering to said patient a single reduced, but effective, amount of a radiopharmaceutical called boronic acid teboroxime-Technetium 99 (BATO-Tc99m) or a derivative thereof, acting as a myocardial radio-labeled imaging agent, wherein the amount of the imaging agent results in reduced radiation exposure for the patient.

In an exemplary method of reducing radiation exposure and total study time to a patient undergoing cardiac diagnosis using a nuclear technique such as single photon emission computed tomography (SPECT) ), especially new SPECT technologies performed with ultrafast gamma-cameras as described below, the method comprises administering to a patient in need thereof a single amount of BATO allowing for the administration of no more than about 5 to about 15 millicuries (mCi) of Tc99m while the patient is under rest conditions, acquiring images of the patient for a period of about 2.5 minutes to about 4 minutes after administration of the imaging agent, administering dipyridamole and adenosine as a slow bolus (stress phase) after about 2.5 minutes to about 4 minutes, and acquiring images of the patient for an additional period of up to about 2 to about 4 minutes, preferably about 2.5 minutes to about 3.5 minutes, after administration of dipyridamole and adenosine.

In an embodiment, the invention relates to a composition, in dry or fluid form, comprising adenosine and dipyridamole in a unit dosage form as defined in the claims, with the unit dosage form being suitable for bolus administration for effecting coronary vasodilation for cardiac diagnosis in a patient in need thereof, where the doses of the dipyridamole and the adenosine are fixed and can be administered independent of the patient's weight. When administered to a patient undergoing cardiac diagnosis using a nuclear technique, the composition allows for a reduction in the amount of the radiopharmaceutical administered to the patient.

In an exemplary method of manufacturing a dry or fluid composition in a unit dosage form, the method comprises combining a fixed dose of adenosine and a fixed dose of dipyridamole in dry or fluid form, into the unit dosage form, such that the unit dosage form is suitable for bolus administration for effecting coronary vasodilation for cardiac diagnosis in a patient in need thereof, and wherein the fixed doses of adenosine and dipyridamole allow for the adenosine and dipyridamole to be administered independent of the patient's weight, and when administered to a patient undergoing cardiac diagnosis using a nuclear technique, allow for a reduction in the amount of the radiopharmaceutical agent administered to the patient.

An exemplary composition, in dry or fluid form, comprises two unit dosage forms, with a first unit dosage form comprising a fixed dose of adenosine and a second unit dosage form comprising a fixed dose of dipyridamole, each of said unit dosage forms being suitable for bolus administration for effecting coronary vasodilation for cardiac diagnosis in a patient in need thereof, and wherein the fixed doses of adenosine and dipyridamole allow for the adenosine and dipyridamole to be administered independent of the patient's weight, and when administered to a patient undergoing cardiac diagnosis using a nuclear technique, allow for a reduction in the amount of the radiopharmaceutical administered to the patient.

In an exemplary method of manufacturing a dry or fluid composition, the method comprises combining a first unit dosage form comprising a fixed dose of adenosine and a second unit dosage form comprising a fixed dose of dipyridamole, each of the unit dosage forms being suitable for bolus administration for effecting coronary vasodilation for cardiac diagnosis in a patient in need thereof, and wherein the fixed doses of adenosine and dipyridamole allow for the adenosine and dipyridamole to be administered independent of the patient's weight, and when administered to a patient undergoing cardiac diagnosis using a nuclear technique, allow for a reduction in the amount of the radiopharmaceutical administered to the patient.

An exemplary kit comprises at least one unit dosage form of dipyridamole and at least one unit dosage form of adenosine, wherein each of the unit dosage forms is suitable for bolus administration for effecting coronary vasodilation for cardiac diagnosis in a patient in need thereof and wherein the fixed dose of adenosine and the fixed dose of dipyridamole allow for the adenosine and dipyridamole to be administered independent of the patient's weight, and when administered to a patient undergoing cardiac diagnosis, allow for a reduction in the amount of imaging agent administered to the patient, and wherein the kit further comprises at least one of a connector, a diluent, an extension set and a venous line.

An exemplary kit can also comprise all the units and elements described in the kit above with in addition a unit dosage form of BATO, or a BATO derivative, allowing for the administration of about 5 mCi to about 15 mCi of Tc99m. The unit dosage form of BATO, or a BATO derivative, and the units of adenosine or dipyridamole or both can be also copackaged.

### BRIEF DESCRIPTION OF THE TABLES

Table 1 describes in the same patient serving as an example, the effect of different combination dose-ratios on mean and peak blood velocities (related to blood flow) measured in resting basal and stress conditions ( in the left descending coronary artery), as well as tolerance: measurements were made before infusion of adenosine 140 µg/kg/min, during a 2 minute infusion of standard adenosine, before injection of the combination bolus (after a 5 min interval at baseline) and after injection of the combination administered as a 30 second IV bolus.

Table 2 describes the mean blood velocities (related to blood flow) in the left descending coronary artery in different series of 3 patients each tested at the same dose ratio of 4 mg adenosine:12 mg dipyridamole in resting basal and stress conditions. Measurements were made before infusion of adenosine 140 µg/kg/min, during infusion of standard adenosine for about 2 minutes, before injection of the combination bolus (after a 5 min interval at baseline) and after injection of the combination given as an IV 30 second bolus.

Table 3 describes the effects of dose ratios of 2.4 mg adenosine: 12 mg dipyridamole and of 2 mg adenosine: 12 mg dipyridamole versus standard adenosine in a series of 12 patients.

### BRIEF DESCRIPTION OF THE DRAWINGS

Fig. 1 illustrates the steps involved in study protocol 1, where administration of the stressor is immediately followed by the imaging agent and continuous imaging over a period of about 5 minutes allows for obtaining information under both stress and resting conditions.
Fig. 2 illustrates the steps involved in study protocol 2, where two doses of the stressor are administered.
Fig. 3 illustrates the steps involved in study protocol 3, where a single injection of a complex of BATO, or a BATO derivative, with a radioisotope is injected under rest conditions, followed after about 2.5 to about 3 minutes by the injection of the stressor and allows for obtaining information under both rest and stress conditions..
Fig. 4 illustrates changes in coronary conductance over time after administration of regadenoson, binodenoson, CGS-21680 (apadenoson-like product), and adenosine.

### DETAILED DESCRIPTION

### 5.1 Overview: Herein are described methods of use, doses, dose ratios, unit dosage forms, kits and compositions.

Functional assessment of myocardial perfusion, also called "coronary reserve assessment," can lead to the detection of myocardial ischemic defects or coronary blood flow insufficiencies, and is important in guiding therapeutic decisions in the care of patients with coronary artery disease. Ischemia is a condition in which blood flow (and thus oxygen) is restricted to a part of the body, often with resultant damage or dysfunction of tissue. Myocardial ischemia, also called cardiac ischemia, occurs when blood flow to the heart muscle is decreased by a partial or complete blockage of an artery that carries blood to the heart. This reduces the ability of the heart to pump efficiently. The decrease in blood flow reduces the supply of oxygen to the heart. A sudden, severe blockage of a coronary artery may lead to a heart attack (myocardial infarction). Myocardial ischemia may also cause serious abnormal heart rhythms. While ischemia is well-correlated to prognosis and to the risks of disease progression, the number of critical stenoses (the abnormal narrowing in a blood vessel) and the degree of narrowing, as detected by coronary angiography, does not appear to correlate with the patient's symptoms, the motion of the muscular walls of the heart, the performance of the heart, the blood flow through the coronary arteries, the patient's prognosis, or with the results of coronary artery bypass surgery. As a consequence, more and more functional tests are now performed during cardiac check-ups^{(1,2)}.

Most current tests for exploring myocardial ischemia status are non-invasive nuclear perfusion imaging methodologies using single photon emission computed tomography (SPECT), projecting a three-dimensional image, with thallium and technetium as the most currently used isotopes. A class of compounds known as BATOs (boronic acid adducts of technetium) has been developed for use in myocardial imaging. Boronic acid teboroxime, also referred to as "BATO", had been approved by the FDA, but has not reached the market and is no longer available for clinical use because current gamma camera detectors are not sensitive enough to detect its signal. However, the arrival of new ultrafast gamma-cameras allow for improved detectability. The development of new semiconductors for gamma photon detection, and in particular the use of cadmium telluride zinc (CZT), is an important breakthrough. It has given rise to the emergence of stationary gamma ray multi-detectors made of CZT modules and to the creation of cameras that reduce SPECT-MP imaging acquisition time. Other ultrafast camera systems are expected to become commercially available (e.g. the avalanche photodiode system using a silicon semiconductor device). Positron emission tomography (PET-scan) using rubidium 82 was recently approved by the FDA for this indication, and is gaining recognition for providing improved images while requiring less exposure to radiation. Nuclear magnetic resonance imaging (MRI) is another technique that may be used to explore myocardial perfusion. Among ultrasound techniques, semi-invasive transeosophageal Doppler Echography is useful to study the motion of ventricular walls, and non-invasive transthoracic Doppler echocardiography is an easy and non-invasive technique for measurement of coronary flow reserve. Coronary flow reserve is the maximum increase in blood flow through the coronary arteries above the normal resting volume. Coronary reserve is the difference between the supply, or flow of blood, in a normal, autoregulated state, and the supply available with maximal vasodilation. The term "coronary reserve dysfunction" relates to a number of conditions in which the coronary reserve is decreased relative to normal values. Another technology, myocardial contrast echography (MCE) uses agents detectable by ultrasound to study myocardium perfusion and heart function in real time with a single test. Yet another imaging technology, X-ray computed tomography (CT) scanning, has been used in studying myocardial perfusion possibly coupled with coronary angiograms. High speed CT scanners, such as ultrafast CT scanners, are capable of taking multiple images of the heart within the time of a single heartbeat. Recently, this technology has been further improved using dual-energy imaging leading to the development of ultra high speed CT scanners.

These functional tests typically require that the patient's heart be "stressed," either through controlled exercise or by pharmacologic means or even both. With technologies such as PET-scanning, MRI or CT scanning, pharmacological agents are the most convenient option to induce stress. With the SPECT technique the two options are possible but there are numerous reasons for choosing pharmacologic stress testing, rather than exercise-induced stress testing. Around thirty percent of patients cannot exercise adequately (due to, for example, peripheral artery disease, left bundle block, aortic aneurysm, obesity or the use of a pace maker) or need special imaging studies to answer specific questions, including whether those with stable angina should be stented, to determine ventricular reserve, to assess responsiveness in assessing patients for valvular surgery, testing of the effectiveness of a therapeutic program in relieving ischemic distress, estimation of the risk of coronary events and cardiac mortality. However, the use of exercise in combination with a stressing agent is becoming more commonly practiced, in particular during SPECT studies.

Adenosine and dipyridamole are two coronary vasodilators used for pharmacologic stress testing ^{(3,4)}. Both of these compounds produce near-maximal coronary dilation through the activity of adenosine. By dilating normal vessels to a greater extent than diseased vessels, these compounds establish a shunt or "myocardial steal" that produces different degrees of increase in flow in healthy versus diseased arteries in patients with coronary artery disease, optimizing the imaging of cardiac muscle areas in need of oxygen supply. Adenosine acts directly by stimulating adenosine purinergic P1 receptors on the arterial wall. These receptors are subdivided into A1, A2a, A2b and A3 receptors. Dipyridamole is believed to work indirectly by blocking the reuptake of adenosine at the cellular level, leading to an increase in endogenous adenosine plasma concentration. Dipyridamole produces similar near-maximal coronary hyperemia (blood flow) to that produced by exogenous adenosine, but it acts less quickly.

Adenosine phosphate derivatives ^{(5,6)}, and more particularly adenosine triphosphate (ATP), have equivalent vasodilating effects as adenosine and can be used for the same purpose. However, since ATP exerts its effect mainly through adenosine, and stimulates both P1 and P2 purino-receptors, ATP has more risks of adverse effects compared to adenosine alone, which acts exclusively on P1 receptors. Therefore, the latter is the preferred molecule and the most commonly used compound.

In current practice, to ensure near-maximal coronary vasodilation and to provide sufficient time for haemodynamic measurements or the acquisition of cardiac images, adenosine must be infused for at least 2 minutes (transthoracic echodoppler study) to 6 minutes (typical SPECT study) at the dosage of 140 µg/kg/min, while dipyridamole must be infused for 4 minutes at the same dosage. Thus, the total recommended dose is 0.84 mg/kg for adenosine and 0.56 mg/kg for dipyridamole. However, in the case of dipyridamole, the total dose can be augmented up to 0.80 mg/kg over 4 minutes and even up to 0.95 mg/kg over 6 minutes if its effect is not considered sufficient ⁽⁴⁾. The average weight of both black and white males between the ages of 35 and 65 is about 80 kg, while the average weight of white females between the ages of 35 and 70 is about 65-70 kg and the average weight of black females between the ages of 35 and 70 is about 75-80 kg. (http://www.halls.md/chart/ height-weight.htm) A person with an average body mass of about 70 kg would therefore receive a total recommended dose of about 58 mg of adenosine or about 39 mg of dipyridamole.

Adenosine is easier to use than dipyridamole due to its immediate effect, while the effect induced by dipyridamole is delayed. For example, when adenosine is used with thallium or technetium scintigraphy, the imaging agent is injected during infusion, about 2 or about 3 minutes after starting infusion, but not at the end of the infusion, because near-maximal vasodilatation is rapidly achieved (after 60 seconds on average). However, when the agent is dipyridamole, the imaging agent is injected about 2 to about 4 minutes following completion of the infusion (typically at about 7 minutes) when the peak pharmacologic effects is reached.

On the side of tolerance, adenosine is responsible for more side effects but due to its very short half-life (less than 10 seconds); the cessation of these side effects is easily obtained by immediate cessation of infusion. In contrast, dipyridamole has a longer half-life with a peak of activity lasting up to 20-30 minutes, which makes adverse reactions last longer, and their management more difficult, this including extra-monitoring time and the frequent use of intravenous aminophylline ⁽⁷⁾. For all these reasons, adenosine is commonly preferred to dipyridamole, at least in North America. ⁽⁸⁾

Although infused during only a few minutes, compounds that stimulate adenosine receptors are accompanied by numerous uncomfortable side effect which are dose-dependent. The most frequently reported adenosine adverse reactions are flushing (44%), chest pain or chest discomfort (40%), dyspnoea (28%), headache (18%), throat or neck or jaw discomfort (15%), and gastrointestinal discomfort (13%). Other side effects are below 10% in incidence ⁽¹⁾.

Adverse effects mostly involve A2a and A1 adenosine receptors and only marginally involve A2b and A3 receptors. For example, flushing, dyspnoea (carotid body stimulation), and hypotension are attributable to A2a receptors, while chest-jaw-neck discomfort or pain, bradycardia and atrio-ventricular blocks are connected with A1 receptors stimulation. Bronchoconstriction is attributable to A2b receptors, but its incidence is very low (7/10,000) (13)

Because dipyridamole is understood to act by increasing endogenous adenosine, use of both adenosine and dipyridamole at full intravenous dosage is contraindicated. Similarly, oral intake of dipyridamole prior to an adenosine pharmacologic stress testing is generally avoided.

U.S. Patent Application No. 11/772,684 (now U.S. Patent No. 7,811,549) and the patent family related to PCT/EP 2007/005923 (now WO-A-2008003479) describe the sequential and concurrent infusion for at least one minute and for a maximum of 6 minutes of adenosine with dipyridamole, where both compounds are used at lower doses than normally applied, for the diagnosis of reversible myocardial ischemic defects. This combination showed that it could maintain optimal coronary vasodilation with a continuous IV infusion while reducing side effects. The recommended dose of adenosine taught in this patent family is 70 µg/kg/min (50% of the standard dose) and the recommended dose of dipyridamole is 10 µg/kg/min (5% of the standard dose), where these compounds are concurrently administered intravenously during 2 to 4 minutes with an electric syringe pump. Another mode of administration can be the bolus administration of dipyridamole 40 µg/kg immediately followed by infusion of adenosine 70 µg/kg/min for the same period of time (2 to 4 minutes) with an electric pump. The duration of action, i.e. near-maximal coronary hyperemia, from the administration of this combination of adenosine and dipyridamole, is very similar to that of the standard dose of adenosine with identical time-to-peak and time to return to baseline. More generally, the patent family discloses a method of inducing coronary vasodilation for use in cardiac diagnosis, the method comprising parenterally administering dipyridamole to said patient; and concurrently or sequentially thereafter parenterally administering adenosine to said patient, wherein dipyridamole is administered intravenously at a total dose of 23-40 µg/kg, and adenosine is administered intravenously at a dosage ratio of about 35 µg/kg/min to about 100 µg/kg/min.

In an effort to reduce side effects at maximally effective agonist doses, adenosinergic agents were, or are being, developed that are selective for the A2a receptor subtype. *See, e.g.,* U.S. Pat. Nos. 6,531,457; 6,448,235; 6,322,771; and 5,877,180. Specific compounds either recently approved by FDA or still in development include regadenoson, binodenoson and apadenoson ⁽¹⁴⁻¹⁹⁾. However, despite increased receptor selectivity, these adenosinergic agents exhibit prolonged and sometimes unpleasant side effects related to their activity on the A2a receptor and/or undue side effects related to their incomplete selectivity or to sympathetic stimulation. The overall reduction of side effects remains modest and sometimes, as is the case for regadenoson, shows an increase in frequency and severity of some adverse events. Regadenoson treatment, for example, results in an increase in dyspnea, headache and gastrointestinal disorders as compared to treatment with the reference drug Adenoscan® (adenosine). These compounds also have a longer duration of action than adenosine (e.g., 10 ± 6 minutes for binodenoson) due to a tighter affinity to the A2a receptor. Accordingly, the A2a-related side effects, e.g., flushing, headache, and dyspnoea, are longer lasting. Thus, although more specific than adenosine, these agents may be more likely to trigger prolonged side effects requiring administration of pharmacologic antidotes, than adenosine itself, whose side effects rapidly dissipate once administration is stopped. Additionally, these products (e.g., regadenoson) may induce direct sympathetic stimulation, in particular an increase in heart rate that is greater than that observed with adenosine. Therefore, the potential risk of ventricular arrhythmia in severe coronary patients should not be underestimated and could pose safety problems in the future.

Nonetheless, a true advantage of A2 agonists is their convenience of use: they can be administered as an IV bolus and at a fixed dose. This simplifies the technique of administration and reduces the risk of dose error.

Most important is the fact that none of the stressing agents recited above (adenosine alone, dipyridamole alone, the combination of adensosine and dipyridamole, and A2 agonists) impacts the level of radiation exposure received by patients during Myocardial Perfusion Imaging (MPI) studies. Currently, the total duration for a MPI-SPECT study including stress and rest protocols, as a rule, ranges from about 4 to about 24 hours and results in radiation exposure to the patient of about 20 to about 30 millisieverts (mSv) and sometimes more. This is considered to be a high dose compared to 8 mSv exposure for a scanner, 2 mSv natural exposure/year for each person, and the total cumulated dose of 100-150 mSv /year, which is considered to be a level at which people are at risk of developing cancer. Due to their kinetic profiles, radiopharmaceuticals commonly used in clinical practice, thallium-201 (T1-201), Tc99m-sestamibi, and Tc99m-tetrofosmin have the disadvantages of requiring time-consuming protocols and of exposing patients to high radiation levels. In this regard, the ideal radio-labeled imaging agent is Tc99m-BATO and other boronic acid adducts of technetium. This is because their kinetic profiles in the heart level are extremely rapid (less than about 4 minutes in total) allowing, in theory, for very short study protocols and low radiation exposure. Unfortunately existing stressors, either because of their mode of administration or long duration effect, do not fit well with Tc99m-BATO's kinetic and ultrafast camera systems. Thus a full stress/rest MPI study with current gamma cameras and marketed stressing agents requires, at the minimum, two isotope injections with an interval of several hours between them. Thus, there is a need to reduce the duration of protocols for the studies and the radiation exposure. Many countries other than the United States, especially European countries, consider radiation exposure as an important issue and a key limiting factor for extensive use of myocardial perfusion scintigraphy in clinical practice. In the United States, higher levels of radiation exposure has been accepted, but this is changing.

Thus, there is still a continuing need in the art for a stressing agent offering the advantages of both adenosine and A2 agonists without their drawbacks. Such an agent would have the following features: convenience of use (no dose adjustment, no electric pump needed), rapid onset of action, maximal coronary dilation maintained for at least about 30 to about 60 seconds but no more than about 90 seconds for optimal efficacy/safety ratio (short duration of action), quick return to baseline (<60 seconds), reduction of side effects, and being adaptable for use in to new techniques allowing for short study protocols and low radiation exposure. The following exemplary embodiments provide such an agent.

The claimed compositions allow for the parenteral bolus injection of adenosine and dipyridamole, at fixed doses which are not based on the patient's weight. A bolus injection involves the administration of a bolus dose of the compound where all of the drug is injected manually in one shot into the vein or artery over a period of about several seconds (a rapid bolus) up to a maximum of about one minute. The term "slow bolus" as used herein refers to the administration of a bolus dose where the compound is administered manually over a period of at least about 20 seconds up to one minute or less. According to the invention, the administration of the bolus occurs over a period of from 20 seconds to 45 seconds. A bolus dose differs from an IV infusion in that in a bolus dose the administration occurs over a short period of time, generally less than about one minute, while an IV infusion takes place over a longer period of time, generally from several minutes to several hours using either an electric syringe or the drip technique. The bolus dosage form has a much higher concentration of the compounds than in the IV infusion, where the compounds are diluted with a carrier, such as an aqueous solution of potassium chloride.

The term "about" means approximately or in the region of. The values encompassed by the number are related to the significant digits in the number. When used in the context of the quantity of a compound, such as "about 3 mg," the weight of the compound could range from 2.5 mg to 3.5 mg, while when used in "about 3.0 mg," the weight of the compound could range from 2.95 mg to 3.05 mg. When used in the context of the concentration of a compound, such as "about 6 mg/ml," the concentration of the compound could range from 5.5 mg/ml to 6.5 mg/ml. When used in the context of the quantity of time, such as "about 45 seconds," the time could range from 42.5 seconds to 47.5 seconds. When the time period is under 10 seconds, the range is ± 2 second.

The use of the terms "from" and "between" in the description of values of ranges include the upper and lower limits.

The terms ultrafast gamma-cameras or ultrafast (MPI) SPECT techniques or ultrafast SPECT cameras as herein means the use of gamma-cameras that have faster imaging acquisition time compared to standard SPECT techniques.

The term "BATO derivatives" means *inter alia* the following compounds:
a boron compound of the formula B-R¹, wherein R¹ is hydroxy, alkyl, alkenyl, cycloalkyl, cycloalkenyl, alkoxy, carboxyalkyl, carboxyalkenyl, hydroxyalkyl, hydroxyalkenyl, alkoxyalkyl, alkoxyalkenyl, haloalkyl, haloalkenyl, aryl, arylalkyl or (R²R³N)-alkyl and R² and R³ are each independently hydrogen, alkyl, or arylalkyl, or R² and R³ when taken together with the nitrogen atom to which they are attached form a 5 or 6-membered nitrogen containing heterocycle.

In an aspect of the disclosure, the parenteral bolus administration is performed in a sequential mode where dipyridamole is administered first as a parenteral bolus injection administered over a period of about 1 to about 5 seconds, immediately followed by a fixed dose of adenosine given as a slow parenteral bolus injection over a period of about 20 seconds to about 45 seconds, preferably about 30 seconds. The efficacy of this mode of administration is not as reliable as the concurrent mode, probably because optimal dipyridamole's effect (inhibition of adenosine capture) on circulating cells requires the drug to act on those cells which are directly in contact with adenosine during the bolus injection and not on the cells that precede adenosine penetration into the vessel. In other words, a simultaneous contact of the two active substances with circulating cells seems to be an important factor for optimal efficacy.

With either sequential or concurrent administration, the total duration of the bolus injection should not be shorter than about 20 seconds or longer than about 45 seconds. Preferred duration of injection is about 30 seconds.

Parenteral injection of the imaging agent can be performed before or after the administration of adenosine and dipyridamole, depending upon study protocol to be performed, as described in various embodiments herein. Injection of the imaging agent itself is generally recommended as about a 10 second bolus.

This mode of administration, using a bolus and a fixed dose of dipyridamole and a fixed dose of adenosine, wherein the doses of dipyridamole and adenosine are independent of the patient's weight, has not been described before for determining coronary reserve or in MPI studies using either adenosine or dipyridamole as stressing agents, since several minutes of infusion are required for each of these products when they are used alone.

Quite surprisingly, it has been found that it is possible to administer concurrently using a bolus to a patient in need thereof a fixed dose of adenosine and dipyridamole where the doses of dipyridamole and adenosine can be administered independent of the patient's weight for the assessment of coronary reserve and in Myocardial Perfusion Imaging (MPI) assessments. As opposed to the usual mode, which requires a continuous infusion during several minutes to maintain efficacy and adjustment of the dose to the patient's weight, it has been discovered that: i) efficacy (maximal coronary hyperemia) with the bolus mode mainly depends on a dose-threshold (about 2 mg adenosine with a minimal dose of 9 mg dipyridamole) rather than on a dose adjusted to the patient's weight, since no dose response is seen beyond this threshold; and ii) at specific dose-ratios beyond this threshold, and regardless of patient weight, the optimal effect can be prolonged by increasing dipyridamole doses, for sufficient time to allow measurements, or imaging assessments without compromising adenosine's capacity to rapidly return to baseline. The fixed dose of adenosine is from 1 mg to 4 mg, preferably 2 mg to 3 mg, more preferably 2.0 mg to 2.5 mg, most preferably 2.1 mg to 2.4 mg, and the fixed dose of dipyridamole is from 9 mg to 14 mg, preferably 12 mg to 14 mg, more preferably 12 mg to 12.6 mg. Optimal efficacy- duration of action/ safety-tolerance ratios are 2 mg to 2.4 mg adenosine combined with 12 mg to 12.6 mg dipyridamole. It should be noted that these dosages, especially for adenosine, are extremely low compared to the total dose of adenosine administered with the reference drug Adenocan® (50 to 90 mg of adenosine/patient depending on the weight). As a result, and as shown in the examples, at specific dose-ratios and following the appropriate protocols described herein, this combination provides efficacy equivalent to that of the reference drug Adenoscan®) for a short, but sufficient, length of time to perform measurements or acquire images with a greater reduction of undue risks (e.g. radiation exposure) than those described in U.S. Patent Application 11/772,684. This also results in improved safety, improved convenience of use and a reduction in the risk of dosing errors.

Doses containing between about 3 mg - about 7 mg adenosine with between about 9 mg - about 14 mg dipyridamole can be efficacious but do not appear to be well tolerated. Such doses could be employed if used in conjunction with sedation, which may no be convenient. Doses of adenosine above about 7 mg with about 9 mg to about 14 mg dipyridamole have a high incidence of 2^{nd} degree atrioventricular blocks and thus are not recommended.

The methods described in this specification can be used to detect a reduced coronary reserve, the presence of any reversible myocardial perfusion defect or myocardial dysfunction using radionuclide imaging techniques (e.g., SPECT, PET-scan) or using ultrasound techniques (TTDE, echocardiography, real time MCE) or MRI and CT-scan imaging techniques.

The methods described herein allow for the drastic reduction in the duration of the SPECT-MPI stress-rest protocols from about several hours to about a few minutes and the reduction by a factor of about 10 of patients' exposure to radiation provided that: i) ultrafast SPECT cameras are used, and ii) the stressor is the combination of adenosine and dipyridamole described herein coupled to radionuclides or radiopharmaceuticals that can be rapidly captured by the myocardium and washed-out from it such as Tc99m-BATO and other boronic acid adducts of technetium. Additionally, it allows for the use of software capable of quantifying Tc99m-BATO's clearance from the heart when used with the adenosine/dipyridamole bolus combination. This makes it possible to quantify almost immediately the severity of myocardial perfusion defects (if any). This information cannot be obtained so rapidly and by a quantitative method using methods currently available.

Under these conditions a single injection of BATO-Tc99m, or a complex of a BATO derivative with Tc99m, is sufficient to achieve a full stress/rest or rest/stress study protocol with a radioactivity dose of at least about 5 mCi (but not less than about 5 mCi to preserve image sensitivity), up to about 15 mCi but no more than this dose to maintain a low radiation exposure. Currently all other existing methods, using either ultrafast or standard cameras, require at least two radiotracer injections to achieve a full stress/rest or rest/stress study protocol with much higher doses injected at rest than under stress conditions. Doses of less than about 5 mCi are not applicable to the method of use described here (which is applicable with a single radiotracer injection).

A hypothetical, but "a posteriori" plausible, understanding of the behavior of adenosine in the blood stream help explain why fixed doses of adenosine and dipyridamole given as a bolus can be administered independent of the patient's weight.

When adenosine is administered by infusion for several minutes (6 minutes recommended at the dose of 140 µg/kg/min), it is circulated throughout the body, going into the lean mass as well as the fat mass. In this type of administration, the body weight (BW) (lean mass + fat mass) of the patient has a strong impact on the dose/adverse events ratio and adjustment of the dose to the weight of the patient is both justified and required. However, when adenosine is administered as a bolus, it is rapidly cleared from the blood stream and most of the effect is limited to the first pass through the lung and the heart, which occurs over a maximum of about 90 seconds in the uses evaluated to date. There is no second pass through the lung and the heart. When administered as a bolus, adenosine is found in a circuit restricted to the veins of the arm, the lung, the heart and the proximal segments of the main arteries close to the heart. In this case, body weight is a poor indicator of the dose/adverse events ratio when red cell volume (between the site of injection and the coronary arteries) becomes the determining factor. Since adenosine is primarily captured by red cells and secondarily by endothelial cells (especially those lining the pulmonary capillaries), red cell volume (RCV) and incidentally functional capillary surface area (FCSA) in the lung are the natural factors that modulate adenosine circulating level and the best variables to predict the side effects of a fixed dose of adenosine given as a bolus in individuals. Of these two, FCSA can be ignored since it is difficult to assess and plays a minor role. RCV is not well related to bodyweight. It has a linear relationship with body surface area (BSA) in women and a slight curvilinear relationship in men (Hurley, Red cell and plasma volumes, J Nucl Med 1974, 16, 46-52*)* and there are several equations relating RCV and BSA. [Two of these equations are: 1100 x BSA (males), 840 x 1.85 (females) and 1550 x BSA-890 (males) and 1167 x BSA-479 (females) - International Committee for standardization in haematology, J Nucl Med, 1980, 21,, 793-800.] These equations show that BSA reflects RCV. However, the circuit described above is limited representing a small part of total BSA so much so that it is nearly impossible to detect dose-effect and dose-adverse event differences, using for example BSA, when adenosine is given as a bolus. Only a threshold is detectable, independently of patients' weight and BSA with no further discrimination above this threshold using these criteria.

Studies to date have found that adenosine doses of about 2 mg to about 3 mg with dipyridamole doses of 9 mg seem correspond to the dose efficacy threshold. Dipyridamole doses of about 12 to about 12.6 mg ensure a duration of the optimal stressing effect of at least about 30 seconds to about 60 seconds on average (not including the non-optimal coronary hyperemia of the landing phase) which is sufficient to perform measurements and acquire images under stress conditions.

Given that adenosine side effects are dose-dependent, that dipyridamole alone used as a 12 mg or 14 mg bolus has no coronary effect and no side effect as mentioned in the examples, that adenosine doses of about 1 to about 4 mg are extremely low dosages compared to those of Adenoscan, the compositions for use in the methods of the present invention reduce the deleterious side effects observed in current practice when adenosine is administered as a single agent at its currently-recommended dosage (e.g., in pharmacological stress testing). Moreover exemplary methods when applied with the appropriate radiotracer and an ultrafast gamma camera, further reduce the radiation exposure incidence and rate of possible serious adverse events related to the use of the adenosine - dipyridamole combination given as a parenteral infusion for more than about one minute. The present invention simplifies the way of administering adenosine. Adenosine triphosphate can be substituted for adenosine, at approximately the same dosage.

The agents described herein can be administered by intravenous or intra-arterial routes to a human.

Convenient unit dosage forms are vials or prefilled syringes containing 1 mg to 4 mg adenosine mixed with 9 mg to 14 mg dipyridamole. Convenient unit dosage forms are vials or prefilled syringes containing 2 mg to 2.4 mg adenosine mixed with 12 mg to 12.6 mg dipyridamole. The amounts of adenosine and dipyridamole can be present in about 0.1 mg amounts within the ranges described above. The pharmaceutical compositions can be dry requiring saline to be added to solubilize them prior to injection. They can be in liquid form comprising carriers and excipients suitable for intravenous or intra-arterial administration, as are well known in the art. Among such excipients are those used in currently approved adenosine compositions, such as saline and mannitol, or those currently approved with dipyridamole, such as tartaric acid, hydrochloric acid and polyethylene glycol (macrogol 60). Hydrochloric acid or other proton donors and sodium hydroxide can be added if necessary for ph adjustment or used instead of tartaric acid.

Unit dosage forms containing the two products mixed together in liquid form have an acid pH of about 2 to about 4.

However in this case, the unit dosage form containing the two products does not require introduction of saline prior to injection, although this is permitted. The mixing of the adenosine-dipyridamole solution with about 2 milliliters to about 3 or 4 milliliters of liquid (including blood) present in the venous line, or/and in the connector, prior to injection, is generally sufficient to buffer the solution. Moreover, in our experience, the slow 30 second injection of the two products at a pH of about 3, directly into the vein through a catheter is immediately buffered by blood and never induced any local side effect or any pain to patients.

In some embodiments, adenosine and dipyridamole are provided in separate unit dosage forms (UDF) and are mixed prior to administration to the patient. These UDFs can be vials or/and prefilled syringes with connection systems permitting the sterile introduction of products from one UDF into the other.

In other embodiments, adenosine and dipyridamole are in separate unit dosage forms and injected sequentially to the patient in less than about 45 seconds (i.e., total time for both injections) as described further above.

The concentration of adenosine is 0.5 mg/ml to 4 mg/ml. Preferably, the concentration of adenosine is 0.5 mg/ml to 1.2 mg/ml. The concentration of dipyridamole is 3 mg/ml to 7 mg/ml more preferably 3 mg/ml, 3.1 mg/ml, 4 mg/ml, 4.1 mg/ml, or 4.2 mg/ml. Most preferred adenosine concentrations are 0.6 mg/ml and 0.8 mg/ml. Most preferred dipyridamole concentrations are 3 mg/ml, 3.1 mg/ml and 4 mg/ml. The concentrations of adenosine and dipyridamole can be in about 0.1 mg/ml incremental amounts within the ranges described above.

The kits comprise at least one unit dosage form of adenosine and at least one unit dosage form of dipyridamole or one unit dosage form containing both products (vial, prefilled syringe) and possibly also a separate unit dosage form of saline acting as a diluent. Adapted connectors and extension set/venous lines are usefully included in the kits.

In some embodiments the kits also contain a separate monodose of BATO, or a BATO derivative, for the administration of a minimal dose of about 5 millicuries and a maximal dose of about 15 millicuries of Tc-99m.

### 5.2 Methods

The methods described above can be used to detect the presence of a reversible myocardial perfusion defect and/or assess the severity of myocardial dysfunction during electrocardiography, echography, echodoppler and/or myocardial perfusion imaging performed by any one of several techniques including the use of a radionuclide agent, regardless of the isotope utilized, such as single photon emission computed tomography (SPECT), positron emission tomography (PET), but also nuclear magnetic resonance (NMR) imaging also called MRI, real time perfusion contrast echocardiography, digital subtraction angiography (DSA), and CT-scan, in particular, ultrafast x-ray computed tomography.

The methods described above can be used with radionuclide angiography (first pass and equilibrium studies utilizing, for example, technetium 99m labeled red blood cells), with any isotope useful for the study of myocardial perfusion, such as thallium-201, technetium sestamibi, technetium tetrofosmine, technetium BATO or a derivative thereof, rubidium 82, nitrogen-13 etc, as well as with any contrast agent used for the same purpose in particular microbubble-based contrast agents (such as perflubutane polymer microspheres, perfluorocarbon, F6 sulphur hexafluoride, and the like).
i. Typically an ultrafast SPECT-MPI sequence with Tc99m-BATO, or a BATO derivative, and the stressing agent, such as the adenosine-dipyridamole bolus combination (ADBC) described in this application requires the patient to stay under the camera during the entire procedure. Exemplary embodiments of three different full (stress/rest) study protocols are provided below. Each of these protocols can be performed in about 6 to about 7 minutes and include a single low dose injection of radiopharmaceutical for the assessment of both stress and rest images. These protocols are not feasible with the use of other stressors and imaging agents. In developing these protocols, the following factors were considered: The duration of action of a stressing agent has a direct impact on the sensitivity of MPI studies to detect myocardial ischemic regions (reversible defects) and on the liver background (its propensity to cause interference with heart images).
*ii.* Sustained coronary vasodilatation accelerates myocardial imaging agent wash-out. This considerably lowers MPI sensitivity since the duration of the contrast between normal and ischemic myocardium is then shortened (a long duration "stress effect" reduces the contrast between healthy and unhealthy regions compared to a short duration stress effect). Having the shortest duration of action, therefore the lowest imaging agent wash-out effect, ADBC is correlated with the highest myocardial sensitivity and thus ADBC is presumed to be the best diagnostic performer.
*iii.* It is also presumed to reduce the capture of Tc99m-BATO, or a BATO derivative, by the liver after about 6 min resulting in reduced liver interference (as opposed to a stressor with a prolonged vasodilatation effect that would increase liver's uptake of BATO).
*iv.* Since the capture of BATO by the liver is reduced with ADBC, no serious liver interference is expected before about 7 to about 8 minutes with this stressor. This allows for continuous stress-imaging acquisition sequences of about 4 minutes to about 5 minutes without the risk of interference and thus does not require the use of Tc99m-BATO at high dosages (about 5 mCi to about 15 mCi, but no more).
*v.* Low doses of BATO-technetium (such as 6 mCi, corresponding to a 1.5 mSv radiation exposure) require longer imaging acquisition times (about 3 to about 4 minutes) compared to a Tc99m-BATO dose of about 9 mCi (corresponding to a 3 mSv radiation exposure) or of about 12 mCi ( about 4 mSv) with acquisition times of about 2 to about 3 minutes.

### Study protocol 1 (continuous stress/rest study)

The steps of this protocol are listed below. The total procedure time for the three phases in protocol 1 is about 5 - about 6 minutes.
1. Injection of ADBC (stressor) typically in about 30 seconds,
2. Injection of Tc99m-BATO, or a BATO derivative, immediately afterwards in less than about 10 seconds,
3. Continuous acquisition of stress images starting about 40 to about 60 seconds after Tc99m-BATO's injection for about 4 to about 5 minutes. Software analysis of Tc99m-BATO' myocardial clearance and of imaging contrast changes is performed at the same time, providing the same information as a full stress-rest protocol.

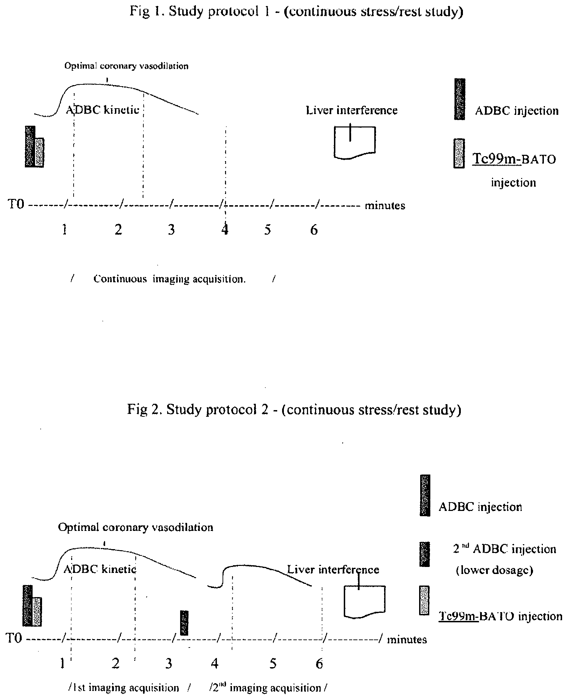

A graphical depiction of the time sequence of the steps of this protocol are shown in Fig. 1.

### Study protocol 2 (continuous stress/rest study)

The steps of this protocol are listed below. The total procedure time for the four phases in protocol 2 is about 6 to about 7 minutes.
1. Injection of ADBC (stressor) in about 30 seconds,
2. Injection of Tc99m-BATO, or a BATO derivative, immediately afterwards in less than about 10 seconds,
3. Continuous acquisition of stress images starting about 40 to about 60 seconds after Tc99m-BATO's injection and continuing for about 3 minutes.
4. Second injection from about minute 3 through about minute 4 (shown at about minute 4) of ADBC at a lower dose followed by about a two-minute acquisition of "redistribution images" (redistribution phase with coronary blood flow going back to baseline = same information as rest images).

A graphical depiction of the time sequence of the steps of this protocol are shown in Fig. 2. This protocol allows for a modification of protocol I that, if necessary, can be implemented by modifying protocol 1 during the execution of protocol 1.

### Study protocol 3 (continuous rest/stress study)

The steps of this protocol are listed below. The total procedure time for the four phases in protocol 3 is about 7 minutes.
1. Injection of Tc99m-BATO, or a BATO derivative, under rest conditions,
2. Continuous acquisition of rest images starting about 40 to about 60 seconds after Tc99m-BATO's injection and for about 2 to about 3.5-4 minutes,
3. Injection of ADBC at from about 2.5 minutes to about 4 minutes (shown at about 3 minutes) without any additional injection of Tc99m-BATO and continuous acquisition of stress images for about 2 to about 3 minutes
4. In this protocol, ischemic regions appear in positive instead of in negative (defect) as opposed to usual protocols

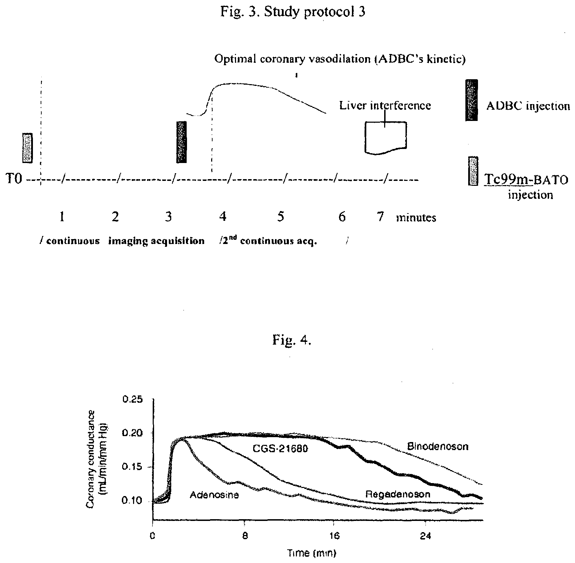

A graphical depiction of the time sequence of the steps of this protocol are shown in Fig. 3. This study mode, which is totally new, is optimal with ADBC because of its ultra-rapid kinetic that enhances contrasts between healthy and non-healthy zones after the uptake of Tc99m-BATO by the myocardium. Again the use of other stressing agents for this rest/stress protocol would result in longer study times and lower imaging sensitivity.

These very short stress/rest protocols are ideally performed with a stressor that is well adapted to the kinetics of Tc99m-BATO, or a BATO derivative. At about 6 minutes after the injection of Tc99m-BATO, the liver captures Tc99m-BATO at a very high concentration. Liver interference usually jeopardizes the acquisition of clean myocardial rest images. This results in the need to perform a separate rest phase study that requires an additional radiopharmaceutical injection. Therefore, the amount of time available for image acquisition under rest conditions following the stress phase must be very short, estimated between about 3 and about 7 minutes after Tc99m-BATO's administration. Among all existing stressors ADBC is the sole product that can perfectly fit with Tc99m-BATO's kinetics, since maximal coronary hyperaemia with ADBC occurs after 30 seconds (post-injection), remains for about 40 to 60 seconds and returns to baseline in about one minute or less.

It is also worth noting that the protocol designs described above cannot be achieved with other stressors either currently commercialized or under development with the exception of ADBC. Time course of changes in coronary conductance caused by the stressors regadenoson, binodenoson, CGS-21680, apadenoson, and adenosine are shown in Fig. 4. These protocols cannot provide the same benefit in terms of reduction of radiation exposure study duration and imaging sensitivity, with Persantine® (dipyridamole) due to its prolonged vasodilating effect and prolonged return to baseline.

The same remark applies to Lexiscan® (regadenoson) as this product induces coronary hyperaemia after 30 seconds. Hyperaemia is maintained for 2 to 3 minutes and is followed by a slow return to baseline, as shown in Fig. 4, meaning that a sustained vasodilating effect persists until about 7 to about 10 minutes post-injection. This requires waiting for a minimum of about 12 to about 15 minute before the acquisition of rest images and requires a second isotope injection. This is too long of a waiting period, with the risk of liver interference (excretion of Tc99m-BATO is enterohepatic, with peak hepatic uptake at about 6 min following injection and there is persistent retention of imaging agent activity within the liver in comparison to technetium) and most importantly of accelerated myocardial imaging agent wash-out that may result in reduced myocardial counts and degraded image sensitivity. Tc99m-BATO's clearance from the myocardium is related to CBF and the shortest clearance is the best. This suggests that a rapid return to baseline (as occurs with the use of ADBC) is an important image sensitivity factor, whereas a prolonged return to base line (Lexiscan®) leads to decreased sensitivity. Sensitivity is the diagnostic determining factor. There is an inverse relationship between affinity and duration of action of A2A agonists. Source: Gao Z et al. J Pharmacol Exp Ther 2001; 298:209-218

Other A2a agonists under development, such as binodenoson and apadenoson, cannot be used because their duration of action (including their return to baseline) is too long.

Adenoscan® (adenosine) (the reference product) cannot be used without modification of its recommended method of use (6 minutes infusion). Adenoscan® would also have the disadvantage of requiring the use of an electric syringe and a dose adjustment to patient's weight.

This shows that ADBC is the ideal stressor for Tc99m- BATO, that the use of Tc99m-BATO with ADBC is the ideal "radio-labeled imaging agent -pharmacological agent combination" for MPI-SPECT ultrafast cameras, and the combination of these three components can be used together to provide methods that can improve imaging processes and reducing exposure to radiation by reducing the amount of imaging agent that needs to be used.

### 5.4 Concentrations and unit dosage forms

In a range of embodiments of the compositions herein provided, the concentration of adenosine is 0.5 mg/ml to 4 mg/ml, preferably from 0.5 mg/ml to 1.2 mg/ml. The concentrations of adenosine may be in about 0.1 mg/ml increments within this range.

In certain embodiments, the concentration of dipyridamole is 3 mg/ml to 7 mg/ml, preferably between 3 mg/ml to 4.2 mg/ml. The concentrations of dipyridamole may be in about 0.1 mg/ml increments within this range.

In various embodiments of compositions comprising adenosine in combination with dipyridamole, the composition has a pH of about 4.0, about 3.9, about 3.8, about 3.7, about 3.6, about 3.5, about 3.4, about 3.3, about 3.2, about 3.1 or about 3.0. In certain embodiments, the pH is between about 2.0 and about 3.0. The term "about" means approximately or in the region of. When used in the context of the pH of the solution, such as "about 3.1," the pH of the solution could range from 3.05 to 3.15.

In some embodiments, the unit dosage form contains about 1 ml to about 10 ml of the pharmaceutical composition formulated as a sterile fluid, typically a sterile, nonpyrogenic, solution suitable for parenteral administration. In some embodiments, the unit dosage form contains about 2 ml, about 3 ml, about 4 ml, about 5 ml, about 6 ml, or about 7 ml. The preferred UDF contains the active substances in about 3 ml or about 4 ml of solution.

In other embodiments, the unit dosage forms of adenosine and dipyridamole are separate with each of them containing, independently of each other, about 1 ml, about 2 ml, about 3 ml, about 4 ml, about 5 ml, about 6 ml, or about 7 ml.

In an embodiment, the unit dosage forms of adenosine and dipyridamole are separate, with one unit dose containing 1 mg to 4 mg of adenosine, preferably 2 mg to 3 mg, more preferably 2.0 mg to 2.5 mg, most preferably 2.1 mg to 2.4 mg and another unit dose containing 9 mg to 14 mg of dipyridamole, preferably 12 mg to 14 mg, more preferably 12 mg to 12.6 mg. In another embodiment, the unit dosage forms of adenosine and dipyridamole are separate, with one unit dose containing 1 mg to 4 mg of adenosine and another unit dose containing 9 mg to 14 mg of dipyridamole.

### 5.5 Stressing agent Kits

Also provided are kits. In exemplary kits, the two agents can be copackaged. For example, in embodiments, the package can usefully contain at least one unit dosage form of dipyridamole copackaged with at least one unit dosage form of adenosine, of an adenosine phosphate, as described above.

In these embodiments, the at least one adenosine and the at least one dipyridamole unit dosage forms are either mixed prior to administration to the patient or injected sequentially. The unit dose of dipyridamole can be packaged in a vial or a pre-packed syringe with or without an injection port and the adenosine unit dose similarly packaged as a vial or a prefilled syringe with or without an injection port, such as a septum, the final objective being to permit sterile introduction of dipyridamole into the adenosine dose or the contrary.

In some kit embodiments, the kit comprises one unit dosage form of the adenosine:dipyridamole composition and possibly also one unit dosage form with saline.

Adapted connectors, diluent (e.g., saline) and extension set/venous lines are usefully included in the kits.

### 5.6 Compositions

In some embodiments, the composition is dry, and suitable for reconstitution prior to injection by addition of a sterile fluid (e.g. saline) into which both dipyridamole and adenosine are readily solubilized. The composition comprises adenosine and dipyridamole in amounts suitable to permit reconstitution in the enclosing vessel to the adenosine and dipyridamole doses above-described.

The pharmaceutical compositions can also be present in separate unit dosage forms, where the amount of adenosine is between 1 mg to 4 mg, preferably 2 mg to 3 mg, more preferably 2.0 mg to 2.5 mg, most preferably 2.1 mg to 2.4 mg and the amount of dipyridamole is 9 mg to 14 mg, preferably 12 mg to 14 mg, more preferably 12 mg to 12.6 mg. These separate dosage forms can be mixed together to form a single composition prior to injection.

### 5.7 Radiopharmaceuticals (imaging agents) and BATO kits

Technetium alone, Thallium alone, are isotopes which are also termed radionuclides. This refers to their capacity to be used as radioactive markers. When isotopes are combined to a carrier that targets a specific organ (sestamibi, tetrofosmine, BATO) the term radiopharmaceutical applies. The term radiopharmaceutical or imaging agent is used herein regardless of this distinction.

Most commonly used radiopharmaceuticals in clinical practice are Thallium-201 (Tl-201), Tc99m-sestamibi and Tc99m-tetrofosmin. Upon injection they are primarily distributed throughout the myocardium in proportion to coronary blood flow (these imaging agents have a high first pass myocardial extraction of over 65%). They are also captured by other tissues but only in a second stage. To facilitate the procedure the initial imaging agent injection is often coupled to the acquisition of images under stress conditions. The acquisition of images at rest then follows. However, this requires the imaging agent, after its initial uptake, to be homogenously redistributed throughout the myocardium, which is not always the case. When it is not the case a delayed session under rest conditions, with a second radiopharmaceutical injection, is needed.

Thallium has a long half-life (73 hrs) and must be given at very low doses to limit radiation exposure over time. This reduces image quality. However, it is well redistributed into the myocardium allowing for the acquisition of images at rest (following those acquired under stress conditions), but not before 4 to 24 hrs after the initial injection, since Tl-201 redistribution is a very slow phenomenon. Even then, a mini dose of thallium is often administered again.

Tc99m-sestamibi and Tc99m-tetrosformin both have a very similar profile. They can be given at higher doses than Thallium and produce better image quality. However, their disadvantage is the lack of redistribution that requires a second radiopharmaceutical injection under rest conditions. This second injection can be done with either thallium or for example Tc99m, but in the latter case at increased dosages.

These radiopharmaceuticals have the disadvantages of requiring time-consuming protocols and expose patients to high radiation levels.

Tc99m-BATO (boronic acid teboroxime) is close to the ideal myocardial perfusion imaging agent and potentially the best of all radiopharmaceuticals. Its extraction fraction (capture by the myocardium) is higher than those of T1-201 or Tc99m. This goes with its excellent correlation to coronary blood flow. Tc99m-BATO's myocardial uptake occurs after only one minute. The imaging agent is redistributed no more than one minute after its capture by myocytes and washed-out from the myocardium after 3 minutes. This rapid uptake and washout, that includes a redistribution phase, allows, in theory, for extremely short stress/rest protocols.

In one aspect of the present disclosure, a unit dosage form comprising an amount of boronic acid teboroxime (BATO) or a derivative allowing for the administration of about 5 millicuries to about 15 millicuries of Tc-99m is provided.

In another aspect, a unit dosage form comprising an amount of boronic acid teboroxime or a derivative allowing for the administration of about 5 millicuries to about 10 millicuries of Tc-99m is provided.

In another aspect, a unit dosage form comprising an amount of boronic acid teboroxime or a derivative allowing for the administration about 6 millicuries to about 12 millicuries of Tc-99m is provided.

In another aspect, a unit dosage form comprising an amount of boronic acid teboroxime or a derivative allowing for the administration of about 10 millicuries to about 15 millicuries of Tc-99m is provided.

In a specific aspect, the unit dosage form of BATO or of a BATO derivative is copackaged with the stressing agent or the two products are in the same kit.

The administration of adenosine at 140 µg/kg/min for a period of 6 minutes is the reference product used in cardiac imaging for inducing near maximal coronary vasodilation. However, the use of the product is accompanied by numerous uncomfortable side effects. In addition to the side effects present, its mode of use requires adjusting the dose to the patient's weights. The method of use also requires the use of an electric infusion pump, which is not convenient. New A2 agonists are more convenient to use (fixed bolus dose) but remain impaired by the persistence of many side effects and a long duration of action. The combined product described in the present application is as efficient as the reference product (adenosine), surprisingly as convenient to use as A2 agonists but additionally has a better safety tolerance profile due to its low dosage and a short duration of action, which is critical with regard to safety. This allows for very short SPECT-MPI stress/rest protocols and reduction of radiation exposure to approximately 1.5-4 mSv/study using ultrafast gamma cameras.

### EXAMPLES

As set forth in these examples, the effects of administering adenosine and dipyridamole intravenously as a bolus and a combined pharmacological stressor, were evaluated versus standard adenosine using transthoracic doppler echocardiography (TTDE) and flow velocity measurements as reflecting blood flow measurements. The value of absolute coronary blood flow (CBF) may be substituted by the value of CBF velocity (peak and mean velocities). This is possible because there is little change in the diameter of epicardial coronary arteries after adenosine administration, the effect of the drug (vasodilatation and coronary steal) being predominant in the intramural microcirculation of the myocardium. Given that blood flow = velocity flow x cross section, if cross section is constant, velocity variations reflect blood flow variations.

TTDE is a non invasive approach conducted under the same conditions as for thallium or technetium scintigraphy (intravenous injection) and another way to study Coronary Flow Reserve (CFR). Thus, it provides more realistic data than those obtained through invasive catheterization laboratory settings. TTDE usefulness to assess CFR has been reported in various clinical studies including more than 1000 patients and has been validated as reproducible and comparable to intracoronary doppler and positron emission tomography (ref 24-27)

The protocol was designed as follows:
- Left anterior descending coronary artery (LAD) velocity measurements (PV and MV) at rest
- Standard adenosine 140 µg/kg/min infusion during 2 minutes.
- LAD velocity measurements during and after 2 minutes of adenosine 140 µg/kg/min infusion.
- Stabilization to baseline during 5 minutes.
- Left anterior descending coronary artery (LAD) velocity measurement at rest
- Bolus injection (30 seconds) of adenosine: dipyridamole at various dosages
- LAD velocity measurement after adenosine:dipyridamole bolus injection
- After each product injection measurements of Time to Peak (TTP), Duration of Optimal effect (DOE), Time to Return to Baseline (RBL),
- Coronary Reserve (CR) calculation
- Assessment of tolerance using specific scales for symptoms, continuous EKG, Blood pressure monitoring

In a dose ranging study still underway, 18 patients were first assessed at adenosine:dipyridamole dose ratios with higher or equivalent dosages of adenosine than dipyridamole such as for example 7 mg to 9 mg of adenosine combined with 6 mg to 9 mg dipyridamole. Results showed that efficacy and in some cases duration of the optimal effect could be reached at cost of an increase of adverse events with occurrence of unacceptable safety issues (3 patients/18 patients with second degree atrioventricular blocks).

The effects of various dosages and ratios of adenosine and dipyridamole administered to patients using several inverted dose-ratios given as about a 30 seconds bolus were evaluated in 25 distinct patients. Different doses ranging from 1 mg to 5 mg of adenosine combined with 12 and 14 mg dipyridamole were repeatedly evaluated. Four tables are shown as examples to illustrate this approach.

### Different adenosine: dipyridamole combinations versus standard adenosine 140µg/kg/min (same as Adenoscan®) given as a 2 minute infusion, in the same patient:

Table 1 shows 5 consecutive studies conducted in the same coronary patient (BW=70 kg, Height=175 cm, BMI=23) each at a one week interval comparing different adenosine-dipyridamole bolus dose-ratios versus standard adenosine (Stad). There was no significant difference between all the doses tested and Stad in terms of efficacy - The efficacy obtained with adenosine 1 mg is slightly lower than that of Stad with a DOE limited to 40 seconds. Best efficacy, duration of optimal effect and coronary reserve results were obtained with the 2 and 2.4 mg adenosine doses, that also induced the highest peak velocities. The 3 mg, 4 mg and 5 mg adenosine doses did not improve (ns) the performance of the combination versus Stad, but clearly increased side effects (scale going from 0 to 10 in severity). It should be noted that side effects with the combination did not last more than 10 seconds which is much shorter than those induced by Stad. No safety issue was observed.

### Effects of the adenosine 4 mg:dipyridamole 12 mg combination

**Table 1: Series of different combinations in the same patient**

| Table 1 | Adenosine (140µg/kg/min) | Ade:dipy 1 mg:12 mg | Ade:dipy 2 mg:12 mg | Ade:dipy 3 mg:12 mg | Ade:dipy 4 mg:12 mg | Ade:dipy 5 mg:12 mg |
|---|---|---|---|---|---|---|
| PV | 23-98 | 24-87 | | | | |
| | 24-105 | | 24-105 | | | |
| | 24-105 | | | 26-110 | | |
| | 37-108 | | | | 31-99 | |
| | 26-96 | | | | | 32-103 |
| MV | 19-78 | 18-68 | | | | |
| | 18-80 | | 17-78 | | | |
| | 18-82 | | | 20-91 | | |
| | 26-75 | | | | 22-72 | |
| | 19-74 | | | | | 25-78 |
| CR (based on MV) | 4.11 | 3.78 | | | | |
| | 4.44 | | 4.59 | | | |
| | 4.56 | | | 4.55 | | |
| | 2.88 | | | | 3.27 | |
| | 3.80 | | | | | 3.12 |
| TTP | 40-50 | 30 | 30 | 30 | 30 | 30 |
| DOE | 60-90 | 40 | 70 | 80 | 75 | 70 |
| RBL | 40-50 | 25 | 25 | 30 | 35 | 30 |
| Toler. | Dyspnoea 2+ to 3+ Flush + | Dyspnoea + | Dyspnoea 2+ Flush + | Dyspneoa 3+ Chest discomfort + | Dyspneoa 4+ | Dyspnoea 6+ |

| | | | | | | |
|---|---|---|---|---|---|---|
| *PV= Peak Velocity (cm*/*sec)-MV=Mean Velocity (cm*/*sec) : first data is at rest, second data is under stress conditions -* *CR =Coronary reserve ratio* *TTP= Time to peak (second)-DOE = Duration of optimal effect (second) -RBL= Return to base line(second) -* *Toler. = Tolerance* | | | | | | |

Table 2 shows the same 4 mg adenosine and 12 mg dipyridamole dose ratio used in three different coronary patients. The 4:12 dose ratio is efficient in all of them and the DOE sufficient. There was no statistical difference between the combination and Stad in terms of efficacy. The only adverse events were dyspnoea and flush. However side effects were not decreased with the combination compared to Stad. No safety issue was observed.

**Table 2. Adenosine 4 mg: dipyridamole 12 mg**

| | St. Adenosine 140µg/kg/min | Patient 1 Ade:dipy 4 mg:12 mg | Patient 2 Ade: dipy 4 mg :12 mg | Patient 3 Ade :dipy 4mg :12 mg |
|---|---|---|---|---|
| MV | 23-50 | 20-57 | | |
| | 14-33 | | 11-32 | |
| | 26-75 | | | |
| | | | | 22-79 |
| CR | 2.12 | 2.85 | | |
| (based on MV) | 2.36 | | 2.91 | |
| | 2.88 | | | 3.12 |
| TIP | | 30 | 35 | 30 |
| DOE | | 55 | 80 | 70 |
| RBL | | 20 | 25 | 30 |
| Tolerance | Dyspnoea : 5+ | Dyspnoea : 3+ | | |
| | Dyspnoea : 3+ | | Dyspnoea : 3+ | |
| | Dyspnoea : 3+ | | | Dyspnoea : 5+ |

Tables 3 and 4 show a series of coronary patients who received either 2 mg adenosine: 12 mg dipyridamole (n=7) or 2.4 mg adenosine : 12 mg dipyridamole (n=10). Efficacy was similar to that of Stad with the 2 mg adenosine combination or even improved while tolerance was better and the number of side effects fewer. The 2.4 mg adenosine dose provided similar results to the 2 mg adenosine dose in terms of efficacy as well as in terms of tolerance. Body weight was not correlated to the results. No safety issue was recorded.

Effects of 2 mg and 2.4 mg adenosine with 12 mg dipyridamole

**Table 3**

| **Dose ratio** | **Adenosine 2 mg:dipyridamole 12 mg** | | | | | | | |
|---|---|---|---|---|---|---|---|---|
| Pt N° | 1 | 2 | 3 | 4 | 5 | 6 | 7 | Mean value |
| Sex | M | M | F | F | M | M | M | |
| BW (Kilogram) | 70 | 98 | 65 | 69 | 80 | 64 | 57 | |
| BMI | 23 | 30 | 25 | 23.5 | 23.4 | 29.5 | 24.4 | |
| TTP | 30 | 30 | 20 | 60 | 40 | 50 | 30 | 37 |
| DOE | 80 | 30 | 50 | 65 | 35 | 45 | 40 | 49 |
| RTBL | 30 | 20 | 30 | 30 | 25 | 35 | 15 | 27 |
| Efficacy vs Stad | = | > | > | < | > | > | < | ns |
| Side effects severity | = | < | < | < | = | < | < | < |

**Table 4**

| **Dose ratio** | **Adenosine 2.4 mg : dipyridamole 12 mg** | | | | | | | | | | |
|---|---|---|---|---|---|---|---|---|---|---|---|
| Pt N° | 8 | 9 | 10 | 11 | 12 | 13 | 14 | 15 | 16 | 17 | Mean value |
| Sex | M | M | M | M | M | M | M | F | M | F | |
| BW (Kilog) | 73 | 84 | 90 | 72 | 74 | 70 | 89 | 84 | 80 | 74 | |
| BMI | 26 | 28 | 29 | 23.5 | 23.4 | 26.3 | 29.4 | 32.6 | 27.7 | 27 | |
| TTP | 30 | 35 | 35 | 50 | 45 | 45 | 35 | 30 | 40 | 40 | 38.5 |
| DOE | 50 | 90 | 45 | 35 | 40 | 40 | 45 | 55 | 45 | 50 | 49.5 |
| RTBL | 30 | 40 | 60 | 15 | 15 | 20 | 25 | 25 | 30 | 30 | 29 |
| Efficacy vs Stad | Efficacy vs Stad | = | = | = | > | = | = | > | = | = | ns |
| Side effects severity | = | > | = | = | = | > | < | < | = | > | ns |

| | | | | | | | | | | | |
|---|---|---|---|---|---|---|---|---|---|---|---|
| *Mean velocity (cm*/*sec): first data is at rest, second data is under stress conditions. CR = Coronary reserve -TTP*= *Time to peak (second)-* *DOE = Duration of optimal effect (second)-* *RTBL= return to base line (second) -* *BW= Body Weight (kilogram)* *BMI=Body Mass Index -[ overweight*= *25-29.9; obesity>30; normal weight=18.5-24.9]* | | | | | | | | | | | |

Additionally a series of 3 patient with 2.1 mg adenosine combined to 12.6 mg dipyridamole and a series of 3 patients with 2.4 mg adenosine combined to 12.4 mg dipyridamole showed very similar results to those described above.

Summary - The following results were also observed:
- Doses as low as 1 mg adenosine (n=3 patients) combined with at least 12 mg dipyridamole can provide as much efficacy as standard adenosine for about 30 to about 40 seconds, in some but not all patients (2 out of three), independently of the patient's weight. This dose is just below and very close to the efficacy threshold.
- Dose ratios of 2 mg adenosine/12 mg dipyridamole and above (e.g. 2.4 mg or 3 mg adenosine: 12 mg dipyridamole) show consistent results which are similar to those of standard adenosine in terms of efficacy, equally efficient and comparable for the duration of the optimal effect (always > 30 seconds) independently of the patient's weight (Tables 1, 3 and 4).
- Dose ratios of 3 mg or 4 mg or 5 mg adenosine/12 mg dipyridamole (n=3 patients /combination) do not further improve efficacy and are not as well tolerated as the 2 mg to 2.4 mg adenosine /12 mg dipyridamole dose-ratios. Tolerance is dose-dependent (Table 2).
- While patients' tolerance of the 2.4 mg adenosine/12 mg dipyridamole dose-ratio and above is comparable to that of Stad given as a 2 minute infusion, the side effects of the combinations are at least two or three times shorter in duration (they do not last more than 10 seconds) than those associated with the administration of Stad.
- Doses of 2.4 mg, (n=10 patients) 3 mg (n=3 patients) and 4 mg (n=3 patients) of adenosine alone administered as a 30 second bolus, are less efficient than Stad. These doses of adenosine alone induce no more than 50 to 60% of the optimal effect induced by Stad and these effects last for only 10 to 15 seconds.
- Dipyridamole, when injected as a bolus alone (n= 5 patients) at the dose of 12 mg, does not induce any significant coronary effect or symptom.
- Dipyridamole mainly serves to modulate the duration of the optimal adenosinergic effect, regardless of the dose of adenosine, provided that dipyridamole is administered at the minimal dose of 9 mg and not above 14 mg. The effect of the dipyridamole dose on mean optimal effect duration is shown below. 14 mg ensures a 60 to 100 seconds duration effect but has the disadvantage to also prolong side effects when they occur.

| Dipyridamole Dose (mg) | Average Optimal Effect Duration (sec) |
|---|---|
| 9 | 30-40 |
| 10 | 40-50 |
| 12 | 40-90 |
| 14 | 60-100 ** |

| | |
|---|---|
| ** While the dose of 14 mg dipyridamole ensures an average optimal effects for about 60 to about 100 seconds, this dose has the disadvantage of prolonging side effects when they occur. | |

Useful dose ratios are summarized in the next paragraph. Ex: 2 mg adenosine/12 mg dipyridamole - 2.1 mg adenosine/12 mg dipyridamole, 2.4 mg adenosine/12 mg dipyridamole etc. Preferred dose ratios are those corresponding to UDFs of 3 and 4 ml.

The table below summarizes all useful and possible unit dosage forms

| Unit dosage form | Adenosine concentration (mg/ml) | Dipyridamole concentration (mg/ml) |
|---|---|---|
| 2 ml | 1, or 1.1 or 1.2 | 6, or 6.1, or 6.2, or 6.3 |
| 3 ml | 0.7 or 0.8 | 4 or 4.1 or 4.2 |
| 4 ml | 0.5 or 0.6 | 3 or 3.1 |

Regardless of the dose injected, base line is reached at about 30 seconds on average after the end of the optimal effect. At 90 seconds after injection no more effect of any sort is observed.

One must understand that beyond the efficacy threshold, corresponding to the minimal number of adenosine receptors recruited to induce coronary hyperemia, efficacy is not improved by the recruitment of additional receptors. However adverse events are then increased. This is why side effects are significantly higher at the dose of 3 mg and 4 mg adenosine/12 mg dipyridamole and above compared to lower doses, while efficacy remains unchanged at these doses compared to lower dose-ratios (such as 2 mg adenosine/12 mg dipyridamole and 2.4 mg adenosine/12 mg dipyridamole).

The same tolerance profile has been observed with regadenoson which is also given as a bolus independently of the patient's weight: 300 µg is the efficacy threshold in many but not all patients, 400 µg is efficient in all patients, 500 µg provides the same efficacy as 400 µg but tolerance is worst. 400 µg is the marketed dose.

### REFERENCES

1. Bateman TM, Prvulovich E - Assessment of prognosis in chronic coronary artery disease. Heart 2004; 90; v10
2. Katritsis DG & Jionnidis JPA - Percutaneous coronary intervention versus conservative therapy in Nonacute coronary artery disease. Circulation 2005, 111, 2906-12.
3. Adenoscan prescribing information (Fujisawa)
4. Injectable Persantin prescribing information (Boehringer Ingelheim)
5. Komukai K, Hashimoto K, Shibata T, Iwano K, Muto M, Mogi J, Imai K, Horie T, Mochizuki S. Effect of continuous ATP injection on human hemodynamics. Circ J. 2002 Oct; 66(10):926-9.
6. Ferreira J, Gil VM, Ventosa A, Calqueiro J, Seabra-Gomes R. Effectiveness and safety of coronary vasodilation with adenosine triphosphate with thallium-201 for the diagnosis of coronary disease. Rev Port Cardiol. 1995 Mar; 14(3):215-24, 188
7. Johnston DL, Daley JR, Hodge DO, Hopfenspirger MR, Gibbons RJ. Hemodynamic responses and adverse effects associated with adenosine and dipyridamole pharmacologic stress testing: a comparison in 2000 patients. Mayo Clin Proc 1995; 70:331-336
8. Taillefer R, Amyot R, Turpin S, Lambert R, Pilon C, Jarry M. Comparison between dipyridamole and adenosine as pharmacologic coronary vasodilators in detection of coronary artery disease with thallium 201 imaging. J Nucl Cardiol. 1996 May-Jun; 3(3):204-11
9. Lagerqvist Bo, Christer Sylvén, Elvar Theodorsen, Lennart Kaijser, Gunnar Helmius and Anders Waldenström - Adenosine induced chest pain - a comparison between intracoronary bolus injection and steady state infusion. Cardiovascular Res, 1992; 26:810-814
10. Crea F, Pupita G, Galassi AR, el-Tamimi H, Kaski JC, Davies G, Maseri A. - Role of adenosine in pathogenesis of anginal pain. Circulation. 1990 Jan; 81(1):164-72
11. Uematsu T, Kozawa O, Matsuno H, Niwa M, Yoshikoshi H, Oh-uchi M, Kohno K, Nagashima S, Kanamaru M. - Pharmacokinetics and tolerability of intravenous infusion of adenosine (SUNY4001) in healthy volunteers. Br.J.Clin.Pharmacol. 2000. Aug; 50(2):177-81.
12. Ross AM, Gibbons RJ, Stone GW, Kloner RA, Alexander RW A randomized, double blinded, placebo-controlled, multicenter trial of adenosine as an adjunct to reperfusion in the treatment of acute myocardial infarction (Amistad-II) - J Am Coll Cardiol, 2005, 45, 1775-80
13. Cerqueira MD, Verani MS, Schwaiger M, Heo J, Iskandrian AS. Safety profile of adenosine stress perfusion imaging: results from the Adenoscan Multicenter Trial Registry. J Am Coll Cardiol. 1994 Feb; 23(2):384-9
14. Cerqueira MD. The future of pharmacologic stress: selective A2A adenosine receptor agonists. Am J Cardiol. 2004 Jul 22; 94(2A):33D-40D; discussion 40D-42D.
15. Iskandrian AE, Bateman TM, Belardinelli L, Blackburn B, Cerqueira MD, Hendel RC, Lieu H, Mahmarian JJ, Olmsted A, Underwood SR, Vitola J, Wang W; ADVANCE MPI Investigators. Adenosine versus regadenoson comparative evaluation in myocardial perfusion imaging: results of the ADVANCE phase 3 multicenter international trial. J Nucl Cardiol. 2007 Sep-Oct; 14(5):645-58.
16. Udelson JE, Heller GV, Wackers FJ, Chai A, Hinchman D, Coleman PS, Dilsizian V, DiCarli M, Hachamovitch R, Johnson JR, Barrett RJ, Gibbons RJ., - Randomized, Controlled Dose-Ranging Study of the selective A2A receptor agonist Binodenoson for pharmacological stress as an adjunct to myocardial perfusion imaging. Circulation 2004; 109:457-64
17. Bristol-Myers Squibb Medical Imaging and Adenosine Therapeutics - Press release March 24, 2004
18. University of Virginia- Induction of pharmacological stress with adenosine receptor agonists. U.S. Pat. No. 6,322,771 and U.S. Patent Application Publication No. 2010/0003193 A1
19. Glover DK, Ruiz M, Takehana K, Petruzella FD, Riou LM, Rigger JM, Macdonald TL, Watson DD, Linden J, Beller GA. Pharmacological stress myocardial perfusion imaging with the potent and selective A(2A) adenosine receptor agonists ATL193 and ATL146e administered by either intravenous infusion or bolus injection. Circulation. 2001 Sep 4; 104(10):1181-7
20. Masaaki Takeuchi et al. - Direct demonstration by Transthoracic Doppler Echocardiography of adenosine-induced coronary steel in the collateral- dependent vessel. Am J Cardiol 2005; 95: 1363-66
21. Di Mario C, MD, PhD; Jeffrey W. Moses, MD; Todd J. Anderson, MD, MRCP; Raoul Bonan, MD; Toshiya Muramatsu, MD; Abnash Chander Jain, MD; Josè Suarez de Lezo, MD; Seung Yun Cho, MD; Morton Kern, MD; Ian T. Meredith, MBBS, PhD; David Cohen, MD, MSc; Issam Moussa, MD; Antonio Colombo, MD; on behalf of the DESTINI Study Group (Doppler Endpoint STenting INternational Investigation) - Randomized comparison of elective stent implantation and coronary balloon angioplasty guided by online quantitative angiography and intracoronary Doppler. DESTINI Study group. Circulation 2000, 103:2938-44
22. Serruys, Patrick W., MD, PhD; Carlo di Mario, MD, PhD; Jan Piek, MD, PhD; Erwin Schroeder, MD; Christian Vrints, MD; Peter Probst, MD, PhD; Bernard de Bruyne, MD; Claude Hanet, MD; Eckart Fleck, MD; Michael Haude, MD; Edoardo Verna, MD; Vasilis Voudris, MD; Herbert Geschwind, MD; Håkan Emanuelsson, MD; V. Mühlberger, MD; Giambattista Danzi, MD; Hans O. Peels, MD; Andrew J. Ford, Jr; Eric Boersma, MSc; ; for the DEBATE Study Group 1 - Prognostic value of intracoronary flow velocity and diameter stenosis in assessing the stenosis and the short and long-term outcomes of coronary balloon angioplasty. The DEBATE Study. Circulation 1997, 96:3369-77
23. Serruys, Patrick W., MD; Bernard de Bruyne, MD; Stéphane Carlier, MD; José Eduardo Sousa, MD; Jan Piek, MD; Toshiya Muramatsu, MD; Chris Vrints, MD; Peter Probst, MD; Ricardo Seabra-Gomes, MD; Ian Simpson, MD; Vasilis Voudris, MD; Olivier Gurné, MD; Nico Pijls, MD; Jorge Belardi, MD; Gerrit-Anne van Es, PhD; Eric Boersma, PhD; Marie-Angèle Morel, MS; Ben van Hout, PhD; on behalf of the Doppler Endpoints Balloon Angioplasty Trial Europe (DEBATE) II Study Group - Randomized comparison of primary stenting and provisional balloon angioplasty guided by flow velocity measurement. Doppler Endpoints Balloon Angioplasty Trial Europe (DEBATE II) Study group. Circulation 2000, 102:2930-37
24. Pizzuto F., MD, Paolo Voci, MD, PhD, Enrica Mariano, MD, Paolo Emilio Puddu, MD, FESC, FACC, Gennaro Sardella, MD and Antonio Nigri, MD - Assessment of flow velocity Reserve by transthoracic doppler echocardiography and venous adenosine infusion before and after left anterior descending coronary artery stenting. J Am Coll Cardiol; 38,(1), 2001, 155-161.
25. Takeshi Hozumi, Kiyoshi Yoshida, Takashi Akasaka, Yoshio Asami, Yumiko Ogata, Tsutomu Takagi, Shuichiro Kaji, Takahiro Kawamoto, Yoshiaki Ueda, and Shigefumi Morioka Noninvasive assessment of coronary flow velocity and coronary flow velocity reserve in the left anterior descending coronary artery by Doppler echocardiography: Comparison with invasive technique. J.Am.Coll.Cardiol.,November 1, 1998; 32(5): 1251-1259.
26. Saraste M, Koskenvuo JW, Knuuti J, Toikka JO, Laine H, Niemi P, Sakuma H, Hartiala JJ. - Coronary flow reserve: measurement with transthoracic Doppler echocardiography is reproducible and comparable with positron emission tomography. Clin Physiol 2001,21:114-22
27. Daimon M., MD, Hiroyuki Watanabe, MD, Hiroyuki Yamagishi, MD, Takashi Muro, MD, Kaname Akioka, MD, Kumiko Hirata, MD, Kazuhide Takeuchi, MD and Junichi Yoshikawa, MD, FACC Physiologic assessment of coronary artery stenosis by coronary flow reserve measurements with transthoracic Doppler echocardiography: comparison with exercise thallium-201 single photon emission computed tomography. J Am Coll Cardiol 2001, 37:1310-15

## Claims

1. A fixed dose of dipyridamole of from 9 mg to 14 mg and a fixed dose of adenosine of from 1 mg to 4 mg, for use in a method of performing cardiac diagnosis, wherein the fixed doses of both dipyridamole and adenosine allow the dipyridamole and adenosine to be administered to the patient independent of the patient's weight, dipyridamole and adenosine being concurrently parenterally injected as a slow bolus over a period of from 20 seconds to 45 seconds.

2. The fixed doses for use according to claim 1, wherein the fixed dose of adenosine is from 2 mg to 3 mg, and the fixed dose of dipyridamole is from 12 mg to 14 mg.

3. The fixed doses for use according to claim 2, wherein the fixed dose of adenosine is from 2.0 mg to 2.4 mg, and the fixed dose of dipyridamole is from 12 mg to 12.6 mg.

4. The fixed doses for use according to any one of the preceding claims, wherein the cardiac diagnosis is used to perform hemodynamic or cardio-electric measurements under stress conditions using transthoracic doppler - echo cardiography or transesophageal doppler echography and/or electrocardiography techniques.

5. The fixed doses for use accordingly to claim 1, wherein an effective amount of imaging agent is administered to said patient.

6. The fixed doses for use according to claim 5, wherein the imaging agent is a radionuclide, a radiopharmaceutical or a contrast agent and the cardiac diagnosis uses a technique selected from the group consisting of SPECT, PET, Doppler, echography, echo-doppler, Nuclear magnetic resonance and CT-scanning technique.

7. The fixed doses for use according to claim 1, wherein dipyridamole and adenosine are administered over a period of 30 seconds.

8. The fixed doses for use according to claim 1, wherein the parenteral administration is selected from the group consisting of intra-arterial administration and intravenous administration.

9. The fixed doses for use according to claim 1, wherein dipyridamole and adenosine are administered intravenously.

10. A composition in dry or fluid form, the composition comprising a fixed dose of adenosine of from 1 mg to 4 mg and a fixed dose of dipyridamole of from 9 mg to 14 mg in a unit dosage form, the unit dosage form being suitable for bolus administration for effecting coronary vasodilation for cardiac diagnosis in a patient in need thereof, wherein the fixed doses of dipyridamole and adenosine allow for administration of the dipyridamole and adenosine independent of the patient's weight.

11. The composition of claim 10, wherein the fixed dose of adenosine is from 2 mg to 3 mg, and the fixed dose of dipyridamole is from 12 mg to 14 mg.

12. The composition of claim 11, wherein the fixed dose of adenosine is from 2.0 mg to 2.4 mg, and the fixed dose of dipyridamole is from 12 mg to 12.6 mg.

13. The composition of claim 11, wherein: (a) adenosine is present in a concentration of 0.5 mg/ml to 4 mg/ml, and (b) dipyridamole is present in a concentration of 3 mg/ml to 7 mg/ml.

14. The composition of claim 13, wherein: (a) adenosine is present in a concentration of from 0.5 mg/ml to 1.2 mg/ml; and (b) dipyridamole is present in a concentration of 3 mg/ml to 4.5 mg/ml.

15. The composition of claim 14, wherein: (a) adenosine is present in a concentration of from 0.6 mg/ml to 0.8 mg/ml; and (b) dipyridamole is present in a concentration of 3 mg/ml to 4.5 mg/ml.

## Patentansprüche

1. Feste Dipyridamol-Dosis von 9 mg bis 14 mg und feste Adenosin-Dosis von 1 mg bis 4 mg zur Verwendung in einem Verfahren zum Stellen einer Herzdiagnose, wobei es die festen Dosen von Dipyridamol und Adenosin ermöglichen, dass Dipyridamol und Adenosin dem Patienten unabhängig von seinem Gewicht verabreicht werden, wobei Dipyridamol und Adenosin gleichzeitig parenteral als ein langsamer Bolus über einen Zeitraum von 20 Sekunden bis 45 Sekunden verabreicht werden.

2. Feste Dosen zur Verwendung nach Anspruch 1, wobei die feste Adenosin-Dosis 2 mg bis 3 mg beträgt und die feste Dipyridamol-Dosis 12 mg bis 14 mg beträgt.

3. Feste Dosen zur Verwendung nach Anspruch 2, wobei die feste Adenosin-Dosis 2,0 mg bis 2,4 mg beträgt und die feste Dipyridamol-Dosis 12 mg bis 12,6 mg beträgt.

4. Feste Dosen zur Verwendung nach einem der vorangehenden Ansprüche, wobei die Herzdiagnose verwendet wird, um hämodynamische oder kardioelektrische Messungen unter Stressbedingungen durchzuführen unter Verwendung von transthorakalen Dopplerechokardiografie- oder transösophagealen Dopplerechografie- und/oder Elektrokardiografietechniken.

5. Feste Dosen zur Verwendung nach Anspruch 1, wobei dem Patienten eine wirksame Menge eines bildgebenden Mittels verabreicht wird.

6. Feste Dosen zur Verwendung nach Anspruch 5, wobei das bildgebende Mittel ein Radionuklid, ein Radiopharmazeutikum oder ein Kontrastmittel ist und die Herzdiagnose eine Technik verwendet, die aus der Gruppe gewählt ist, die aus SPECT, PET, Doppler, Echografie, Echo-Doppler, Kernspinresonanz und CT-Scantechnik besteht.

7. Feste Dosen zur Verwendung nach Anspruch 1, wobei Dipyridamol und Adenosin über einen Zeitraum von 30 Sekunden verabreicht werden.

8. Feste Dosen zur Verwendung nach Anspruch 1, wobei die parenterale Verabreichung aus der Gruppe gewählt ist, die aus intraarterieller Verabreichung und intravenöser Verabreichung besteht.

9. Feste Dosen zur Verwendung nach Anspruch 1, wobei Dipyridamol und Adenosin intravenös verabreicht werden.

10. Zusammensetzung in trockener oder fluider Form, wobei die Zusammensetzung eine feste Adenosin-Dosis von 1 mg bis 4 mg und eine feste Dipyridamol-Dosis von 9 mg bis 14 mg in einer Einzeldosisform umfasst, wobei die Einzeldosisform geeignet ist zur Bolusverabreichung zum Erzielen von Koronargefäßerweiterung zur Herzdiagnose in einem Patienten, der diese benötigt, wobei die festen Dipyridamol- und Adenosin-Dosen eine Verabreichung des Dipyridamols und Adenosins unabhängig von dem Gewicht des Patienten ermöglichen.

11. Zusammensetzung nach Anspruch 10, wobei die feste Adenosin-Dosis 2 mg bis 3 mg beträgt und die feste Dipyridamol-Dosis 12 mg bis 14 mg beträgt.

12. Zusammensetzung nach Anspruch 11, wobei die feste Adenosin-Dosis 2,0 mg bis 2,4 mg beträgt und die feste Dipyridamol-Dosis 12 mg bis 12,6 mg beträgt.

13. Zusammensetzung nach Anspruch 11, wobei: (a) Adenosin in einer Konzentration von 0,5 mg/ml bis 4 mg/ml vorliegt und (b) Dipyridamol in einer Konzentration von 3 mg/ml bis 7 mg/ml vorliegt.

14. Zusammensetzung nach Anspruch 13, wobei: (a) Adenosin in einer Konzentration von 0,5 mg/ml bis 1,2 mg/ml vorliegt und (b) Dipyridamol in einer Konzentration von 3 mg/ml bis 4,5 mg/ml vorliegt.

15. Zusammensetzung nach Anspruch 14, wobei: (a) Adenosin in einer Konzentration von 0,6 mg/ml bis 0,8 mg/ml vorliegt und (b) Dipyridamol in einer Konzentration von 3 mg/ml bis 4,5 mg/ml vorliegt.

## Revendications

1. Dose fixe de dipyridamole de 9 mg à 14 mg et dose fixe d'adénosine de 1 mg à 4 mg, en vue d'une utilisation dans un procédé de réalisation de diagnostic cardiaque, les doses fixes à la fois de dipyridamole et d'adénosine permettant au dipyridamole et à l'adénosine d'être administrés au patient indépendamment du poids du patient, le dipyridamole et l'adénosine étant injectés simultanément par voie parentérale sous forme de bolus lent sur une période de 20 à 45 secondes.

2. Doses fixes en vue d'une utilisation selon la revendication 1, dans lesquelles la dose fixe d'adénosine va de 2 mg à 3 mg et la dose fixe de dipyridamole va de 12 mg à 14 mg.

3. Doses fixes en vue d'une utilisation selon la revendication 2, dans lesquelles la dose fixe d'adénosine va de 2 mg à 2,4 mg et la dose fixe de dipyridamole va de 12 mg à 12,6 mg.

4. Doses fixes en vue d'une utilisation selon l'une quelconque des revendications précédentes, dans lesquelles le diagnostic cardiaque est utilisé pour réaliser des mesures hémodynamiques ou cardio-électriques dans des conditions de stress par le biais d'un Doppler transthoracique - échocardiographie ou échographie Doppler transoesophagienne et/ou techniques électrocardiographiques.

5. Doses fixes en vue d'une utilisation selon la revendication 1, dans lesquelles une quantité efficace d'agent d'imagerie est administrée audit patient.

6. Doses fixes en vue d'une utilisation selon la revendication 5, dans lesquelles l'agent d'imagerie est un radionucléide, un agent radiopharmaceutique ou de contraste et le diagnostic cardiaque utilise une technique choisie dans le groupe constitué par SPECT, PET, Doppler, échographie, écho-Doppler, résonance magnétique nucléaire et technique de tomographie.

7. Doses fixes en vue d'une utilisation selon la revendication 1, dans lesquelles le dipyridamole et l'adénosine sont administrés sur une période de 30 secondes.

8. Doses fixes en vue d'une utilisation selon la revendication 1, dans lesquelles l'administration parentérale est choisie dans le groupe constitué par une administration intra-artérielle et une administration intraveineuse.

9. Doses fixes en vue d'une utilisation selon la revendication 1, dans lesquelles le dipyridamole et l'adénosine sont administrés par voie intraveineuse.

10. Composition sous forme sèche ou liquide, la composition comprenant une dose fixe d'adénosine de 1 à 4 mg et une dose fixe de dipyridamole de 9 à 14 mg sous forme de dosage unitaire, la forme de dosage unitaire étant adaptée à une administration de bolus destinée à effectuer la vasodilatation coronarienne pour le diagnostic cardiaque chez un patient qui en a besoin, les doses fixes de dipyridamole et d'adénosine permettant l'administration du dipyridamole et de l'adénosine indépendamment du poids du patient.

11. Composition selon la revendication 10, dans laquelle la dose fixe d'adénosine va de 2 à 3 mg et la dose fixe de dipyridamole va de 12 mg à 14 mg.

12. Composition selon la revendication 11, dans laquelle la dose fixe d'adénosine va de 2,0 mg à 2,4 mg et la dose fixe de dipyridamole va de 12 mg à 12,6 mg.

13. Composition selon la revendication 11, dans laquelle : (a) l'adénosine est présente en une concentration de 0,5 mg/ml à 4 mg/ml et (b) le dipyridamole est présent en une concentration de 3 mg/ml à 7 mg/ml.

14. Composition selon la revendication 13, dans laquelle : (a) l'adénosine est présente en une concentration de 0,5 mg/ml à 1,2 mg/ml ; et (b) le dipyridamole est présent en une concentration de 3 mg/ml à 4,5 mg/ml.

15. Composition selon la revendication 14, dans laquelle : (a) l'adénosine est présente en une concentration de 0,6 mg/ml à 0,8 mg/ml ; et (b) le dipyridamole est présent en une concentration de 3 mg/ml à 4,5 mg/ml.
